# EUROPÄISCHE PATENTANMELDUNG

(11) **EP 0 629 379 A1**
(43) Veröffentlichungstag der Anmeldung: **21.12.1994**
(21) Anmeldenummer: 93108759.7
(22) Anmeldetag: 01.06.1993
(51) Int. Cl.: A61B 17/22

(54) **Vorrichtung zum Ablösen von sklerotischen Plaques mittels Ultraschall**

(71) Anmelder: La Rosa, Antonio, I-27100 Pavia (IT)
(72) Erfinder: La Rosa, Antonio, I-27100 Pavia (IT)
(74) Vertreter: Kaminski, Susanne, Dr.

(57) **Zusammenfassung**

Eine mit einem an einen Ultraschall-Generator (17) anschließbaren Ultraschall-Transmitter versehene Vorrichtung zur Ablösung von aterosklerotischen Plaques mittels Ultraschall-Schwingungen weist eine an einem Handgriff (1) befestigte Sonde (7;8) auf. Die Sonde (7;8) besteht aus einem im wesentlichen transversal schwingenden Arbeitsteil und einem im wesentlichen longitudinal schwingenden Übertragungsteil. Der Übertragungsteil ist von einem Flüssigkeit führenden Röhrchen vollständig umhüllt, welche Flüssigkeit zur Benetzung des Arbeitsteils der Sonde (7;8) dient, wobei für die Zuführung der Flüssigkeit in das Röhrchen ein Kanal im Handgriff (1) vorgesehen ist. Das Röhrchen besteht aus einem Material, dessen Reibungskoeffizient gegenüber der Flüssigkeit vernachlässigbar ist und es sind Mittel zum allseitigen Benetzen des Arbeitsteils mit Flüssigkeit vorgesehen sind.

## Beschreibung

Die Erfindung betrifft eine Vorrichtung zur Ablösung von sklerotischen Plaques nach dem Oberbegriff des Anspruchs 1 und ein Behandlungsset, das eine solche Vorrichtung enthält, nach dem Oberbegriff des Anspruchs 9.

Die Entfernung von arteriellen Obturationen bzw. Okklusionen, die auf die Bildung von aterosklerotischen Plaques zurückgehen, mit Hilfe von Ultraschall ist eine von mehreren, gebräuchlichen Methoden.

Die klassische Endarteriektomie, bei der der am Verschlußort der Arterie gelegene Thrombus einschließlich der ihm anhaftenden Innenwand der Arterie, der Intima und gegebenenfalls auch der Media, ausgeräumt wird, erfolgt entweder als sogenannte "offene Endarteriektomie", wobei die Arterie der Länge nach geöffnet wird, oder als sogenannte "halboffene Endarteriektomie" von zwei Querschnitten in der Arterie aus, mittels sogenannter Ringstripper. Ist zwar bei der offenen Endarteriektomie der Vorteil gegeben, das Ausräumen des Thrombus visuell überwachen zu können, so sind doch die Nahtpunkte der wiederhergestellten Arterie mögliche Ursache von Stenosen. Bei der halboffenen Endarteriektomie hingegen liegt das Problem darin, daß - insbesondere bei länger ausgedehnten Obstruktionen - das mechanisch erfolgende Ausräumen mittels eines Strippers vielfach unregelmäßige Arterien-Innenwandoberflächen zur Folge hat. Ist aber die Media nur teilweise entfernt, oder wurde sie verletzt, so führt das in den meisten Fällen zu postoperativen Hyperplasien.

Bei der Angioplastie werden okkludierte Arterien mit Hilfe eines Ballonkatheters aufgedehnt. Diese Behandlung, die vielfach als vorbereitende bzw. zusätzliche Maßnahme zur Gefäßplastik vorgenommen wird, wirkt jedoch immer in einem gewissen Maße traumatisierend. Sekundär-Stenosen aufgrund von miointimalen Hyperplasien sind die Folge.

Plaques relativ geringer Ausdehnung werden auch mit Hilfe von Lasern aufgelöst; es besteht das Risiko thermischer Reizung bzw. Verletzung der Gefäßinnenwand, sogar Gefäßperforationen sind möglich.

Auch die Verwendung von Ultraschall zur Entfernung von aterosklerotischen Plaques wurde vorgeschlagen, so beispielsweise in der US-3,565 062, in der eine hohle, schwingende Sonde beschrieben ist, wobei ein dieser Sonde zugeordneter, mit Schlitzen versehener Katheter von der Gefäß-Innenwand abgeschnittene Plaque-Streifen aufnimmt. In der US-3,526 219 wird die Entfernung von Gewebeteilen beschrieben, wobei die Ultraschall-Energie dazu verwendet wird, das Gewebe in kleinste Teile zu zerteilen ("micro-chopping"), die dann abgesaugt werden können. In beiden Fällen - wie übrigens für die in der US-3,526 219 beschriebene Methode bereits in der US-4,870 953 festgestellt - können einerseits traumatisierende Einwirkungen auf die Media erfolgen bzw. besteht andererseits Unsicherheit über den tatsächlichen Umfang der entfernten Okklusionen.

In der US-4,962 755 wird eine Methode beschrieben, bei der eine kurze, gebogene Ulraschall-Sonde in einen Einschnitt in der Arterie eingebracht wird. Dem vibrierenden Arbeitsteil der Sonde wird Flüssigkeit über ein starres, parallel zur Sonde angeordnetes Röhrchen zugeführt. Da einerseits der vibrierende Arbeitsteil zwischen abgelöster Plaque bzw. Media und Adventitia zu liegen kommt und andererseits bei weiterem Einschieben in die Arterie das Flüssigkeitsröhrchen nicht mit eingeschoben wird, ist die Benetzung des vibrierenden Arbeitsteils nur einseitig und das auch nur bedingt möglich. Damit wird nach Lockerung der Plaque von einem in der Mitte der Okklusion vorgenommenen Einschnitt aus bzw. von zwei distal bzw. proximal vorgenommenen Einschnitten aus die Entfernung der Plaques mittels Forceps nötig. Es ist offensichtlich, daß eine solche Methode nur bei sehr kleinflächigen Okklusionen erfolgreich sein kann, da ansonsten bei der Entfernung der gegebenenfalls nur teilweise gelösten Plaques mittels Forceps ein Losreißen derselben von der Media bzw. Zerreißen und damit nur unvollständiges Entfernen derselben die Folge sein kann. Traumatisierung der Media ist nicht auszuschließen.

Dazu kommt, daß auch hier das Problem des einwandfreien Präparierens des "end-point"nicht gelöst wird. Dieses Problem, das bereits in der klassischen Endarteriektomie nicht befriedigend gelöst werden konnte, besteht darin, daß der Übergang zwischen der behandelten Arterien-Innenwand und jenem Bereich der Arterien-Innenwand, in dem unbehandelte Intima und Media verbleiben, homogen und regelmäßig sein muß, da andernfalls dies eine der möglichen Ursachen für postoperative Thrombosen ist.

In der AT-PS-340 572 werden Ultraschall-Sonden beschrieben, die für die Entfernung sowohl von kleinflächigen als auch von ausgedehnten Plaques gedacht sind. Bei der kurzen, geknickten Sonde, die ähnlich der in der US-4,962 755 beschriebenen ausgebildet ist, wird die Flüssigkeit, als eine von zwei beschriebenen, alternativen Möglichkeiten, über einen im Inneren der Sonde vorgesehenen Kanal durch an der Übergangsstelle zum geneigten Teil vorgesehene Austrittsöffnungen dem schwingenden Arbeitsteil zugeführt. Da der Arbeitsteil aber transversal schwingt, wird die Flüssigkeit nicht bis zur Spitze des Arbeitsteils gelangen können, sondern bereits vorher abgespritzt werden. Auch bei der langen Sonde, die in Form eines Ringstrippers ausgebildet ist, wird die Flüssigkeit - wiederum als eine von zwei alternativen Möglichkeiten - über einen in der Sonde vorgesehenen Kanal dem transversal schwingenden Ring zugeführt. Abgesehen von der wiederum unzureichenden Benetzung des Ringes wird die Wirksamkeit der langen Sonde während ihrem Einschieben in das Innere einer Arterie zunehmend verringert werden, da der den transversal schwingenden Ring tragende, einen inneren Kanal aufweisende, lange Stab sich in dem engen Zwischenraum zwischen Adventitia und abgelöster Plaque bzw. abgelöster Media vorwärtsschiebt. Damit wird auf den longitudinal schwingenden Stab von Gefäß-Innenwand und abgelösten Schichten ein Druck ausgeübt, der einen signifikanten Abfall der Wirksamkeit der Sonde zur Folge hat. Letztlich, da Knoten gebildet werden, sind keinerlei Transversalschwingungen des Ringes mehr gegeben.

Die Erfindung hat sich demgegenüber die Aufgabe gestellt, eine Vorrichtung bereitzustellen, die das Ablösen von sklerotischen Plaques und der Media, und zwar unabhängig vom Grad der aterosklerotischen Beeinträchtigung derselben, bei - abgesehen von einem kleinen Einschnitt - geschlossener Arterie ermöglicht, wobei traumatisierende Einwirkungen auf die übrigbleibende Gefäßwand vermieden werden.

Dies gelingt durch die Verwirklichung der kennzeichnenden Merkmale des Anspruchs 1.

Vorteilhafte Weiterbildungen werden durch die kennzeichnenden Merkmale der abhängigen Ansprüche beschrieben.

Dadurch, daß Mittel vorgesehen sind, die es ermöglichen, daß der - im allgemeinen in transversale Schwingungen versetzte - Arbeitsteil über seine ganze Oberfläche mit Flüssigkeit benetzt wird, die über das den Übertragungsteil, der im wesentlichen in longitudinale Schwingungen versetzt wird, vollständig umhüllende Röhrchen zugeführt wird, wird die - im wesentlichen durch das Auftreten von Kavitation bedingte - Effektivität der Sonde beim kompletten Lösen der Media (und der Plaque) von der Adventitia verbessert. Außerdem ist dadurch, daß das den Übertragungsteil umhüllende Röhrchen aus einem Material ist, dessen Reibungskoeffizient gegenüber der Flüssigkeit vernachlässigbar ist, die Möglichkeit gegeben, den Übertragungsteil vor Energieverlusten zu schützen, die sich insbesondere bei direktem Kontakt mit der Arterieninnenwand ergeben würden, da es zu keiner negativen Rückkopplung auf Übertragungs- und damit Arbeitsteil der Sonde durch Übertragung von Schwingungen bzw. Bildung von Schwingungsknoten kommt.

Ist der Arbeitsteil der Sonde gegenüber der Längsachse des Übertragungsteils abgewinkelt ausgebildet - wobei die spezifische Gestaltung des Arbeitsteils von der notwendigen Behandlung, wie Art und Lumen der Arterie und Ausdehnung der Plaque, abhängen wird - so wird der Zugang zum Inneren der Arterie vereinfacht.

Ein spatelförmig ausgebildeter Arbeitsteil bietet sich für das Ablösen von kleinflächigen Plaques an, was gegebenenfalls von zwei Einschnitten in der Arterie aus geschehen kann, die distal und proximal vorgenommen werden. Ein derart ausgebildeter Arbeitsteil wird auch zur Präparierung des distalen, sogenannten "end point" verwendet, bei dem die Media von der Adventitia gelöst wird, wonach eine anders ausgebildete Sonde durch den kleinen Einschnitt in der Arterie eingeführt werden kann. Eine solche Sonde, deren Arbeitsteil in Form eines Ringes, der Übertragungsteil jedoch als langer, dünner Stab ausgebildet ist - in Art der aus der klassischen, halboffenen Endarteriektomie bekannten Ringstripper - , erweist sich für das Entfernen von ausgedehnten Obliterationen, beispielsweise in der Arteria femoralis, von Vorteil.

Wie bereits einleitend dargestellt, sind an sich ähnliche Ausbildungen solcher Sonden für die Verwendung bei der klassischen Endarteriektomie und auch bei Sonden, die mit Ultraschall arbeiten, bekannt. Aber sowohl bei der klassischen Endarteriektomie als auch bei einer Ablösung der Plaques mit Hilfe von Ultraschall mittels einer in Art der in der AT-A-340 572 beschriebenen Sonde sind traumatische Einwirkungen nicht auszuschließen, ebensowenig wie unvollständiges Entfernen der aterosklerotisch lädierten Media.

Die erfindungsgemäße Sonde zeichnet sich demgegenüber dadurch aus, daß sie die - es wird mit relativ niedrigen Frequenzen um die 30kHz gearbeitet - Freilegung des Verschlußbereichs entlang der gesamten Länge der Arterie bei schonender, d.h. nicht traumatisierender, und dabei vollständiger Ablösung der Media von der Adventitia ermöglicht, da selbst dann, wenn die Sonde weit in die Arterie eingeschoben ist, der ringförmige Arbeitsteil mit im wesentlichen unveränderter Energie und Frequenz schwingt und gleichzeitig über seine Ringfläche gleichmäßig mit Flüssigkeit benetzt wird.

Der Übertragungsteil einer solchen, im folgenden als Ringsonde bezeichneten Sonde, ist vorzugsweise als dünner Stahlstab mit einem Durchmesser von ca 1 mm bei einer Länge von bis zu 60 cm und mehr ausgebildet, so daß er auch bei leicht gekrümmten Arterienverläufen widerstandsfrei geführt werden kann. Das die Flüssigkeit führende, den Übertragungsteil umhüllende Röhrchen ist vorzugsweise aus einem formfesten, jedoch über seine Längserstreckung biegsamen Kunststoff geformt, für den sich insbesondere Polytetrafluorethylene (PTFE) aufgrund seiner ausgezeichneten, antiadhäsiven Eigenschaften anbietet. Damit wird sowohl eine verbesserte Gleitfähigkeit der Sonde in dem Zwischenraum zwischen abgelöster Media und Adventitia erreicht, als auch ein inertes Verhalten gegenüber gegebenenfalls über die Flüssigkeit übertragbare Schwingungen.

Damit der ringförmige Arbeitsteil der Ringsonde über seine gesamte Ringfläche mit Flüssigkeit benetzt wird, wird die Flüssigkeit über eine ringförmige Öffnung, die gegebenenfalls durch den Durchmesser des Röhrchens selbst gegeben ist, ausgebracht. Da die Flüssigkeit mit Druck aus dieser Öffnung austritt, wird sie beidseitig der Ansatzstelle zwischen Ring und Übertragungsteil auf den schwingenden Ring verteilt.

Die Sonde mit dem spatelförmigen Arbeitsteil, im folgenden als Spatelsonde bezeichnet, kann - in bezug auf Übertragungsteil und Röhrchen für die Zuführung der Flüssigkeit - prinzipiell entsprechend der Ringsonde ausgebildet sein. Da jedoch diese Sonde im allgemeinen zur Behandlung eines unmittelbar an den Einschnitt in die Arterie angrenzenden Bereichs eingesetzt wird, wobei ausschließlich deren Arbeitsteil in die Arterie eingeschoben wird, muß der Frage der Gleitfähigkeit des Röhrchenmaterials und der Steifigkeit und Biegsamkeit desselben weniger Augenmerk geschenkt werden. Dazu bietet sich z.B. auch ein Material wie PVC an, das einfacher und kostengünstiger zu verarbeiten ist, bei Wahrung ähnlich vorteilhafter Eigenschaften in bezug auf die Vermeidung von Energieverlusten.

Die Ausbildung des Arbeitsteils in Form eines Spatels ist zwar bevorzugt, da damit die Führung zwischen abgelöster Media und Adventitia vereinfacht ist, jedoch sind auch andere Ausgestaltungen denkbar, beispielsweise stäbchenartige oder linsenkopfförmige. Das die Flüssigkeit führende Röhrchen kann den spatelförmigen Arbeitsteil im Bereich des Übergangs zum Übertragungsteil vollständig umschließen, was sich insbesondere dann anbieten wird, wenn es aus relativ weichem Material gefertigt ist. Für den Auslaß der Flüssigkeit sollten dann wenigstens zwei Öffnungen vorgesehen sein, die zu beiden Seiten des Spatels angeordnet sind, so daß die mit einem bestimmten Druck austretende Flüssigkeit über die gesamte Fläche des Arbeitsteils verteilt wird. In gleicher Weise können mehrere Öffnungen vorgesehen sein, jedenfalls symmetrisch um den Umfang des beliebig ausgebildeten Arbeitsteils.

Ein Behandlungsset, das den raschen, behandlungsspezifischen Wechsel der Behandlungssonden während einer Behandlung ermöglicht, wird in vorteilhafter Weise den Behandlungsablauf vereinfachen und optimieren.

Die Erfindung wird im folgenden anhand einer Zeichnung beispielhaft beschrieben. Es zeigen:
- Fig.1: einen Ultraschall-Generator, an den zwei Vorrichtungen zum Ablösen von aterosklerotischen Plaques angeschlossen sind;
- Fig.2: eine zum Einführen ins Innere einer Arterie geeignete Sonde;
- Fig.3: eine zum Lösen von kleinflächigen Plaques geeignete Sonde;
- Fig.3a: die Sonde der Fig.3, unter einem anderen Blickwinkel gesehen und
- Fig.4a bis 4l: aufeinanderfolgende Schritte des Ablösevorgangs der einer langausgehnten Okklusion in der Arteria femoralis.

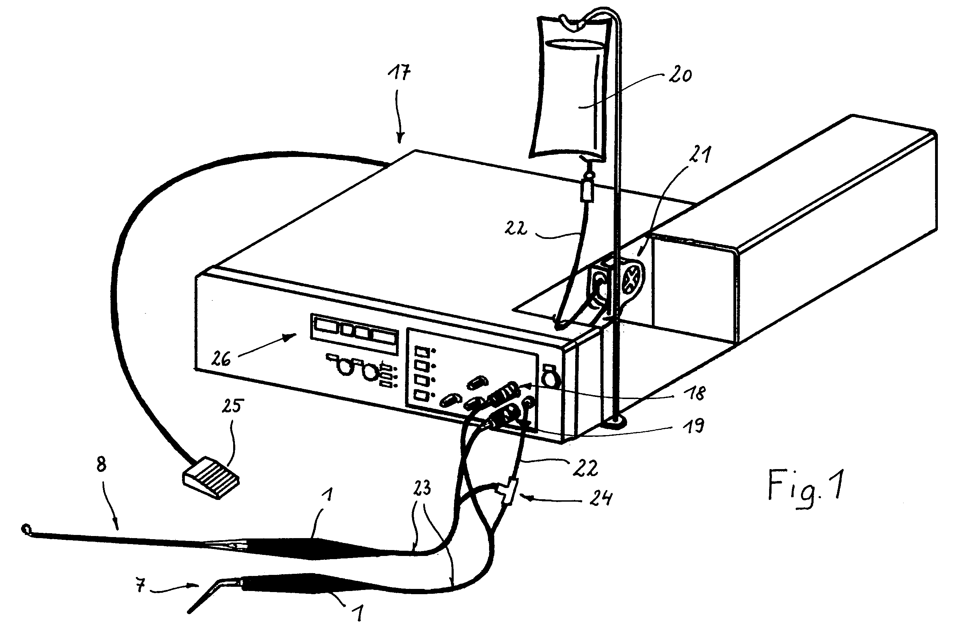

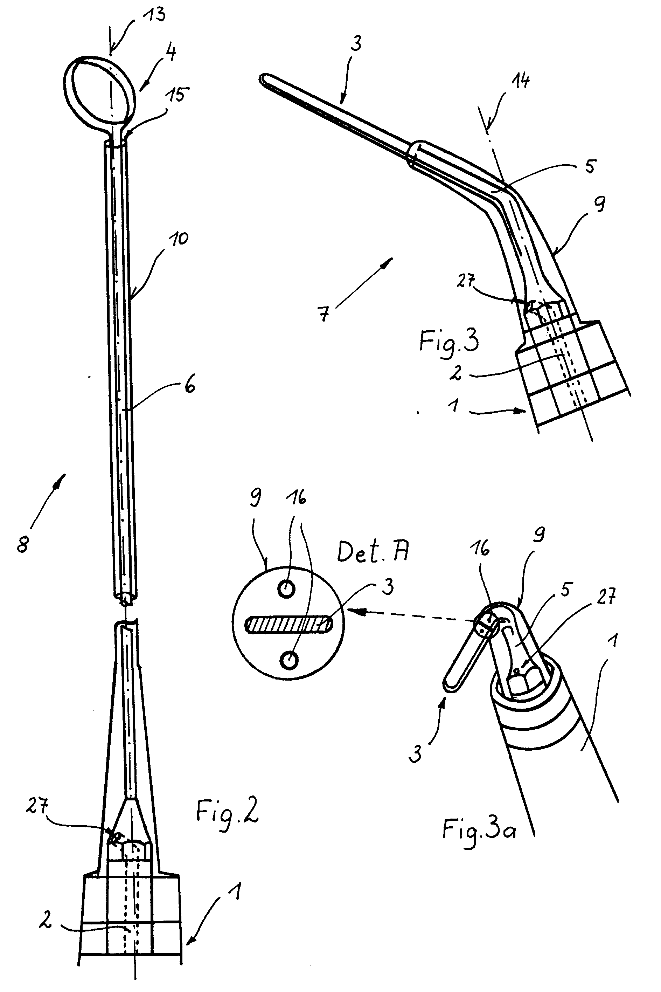

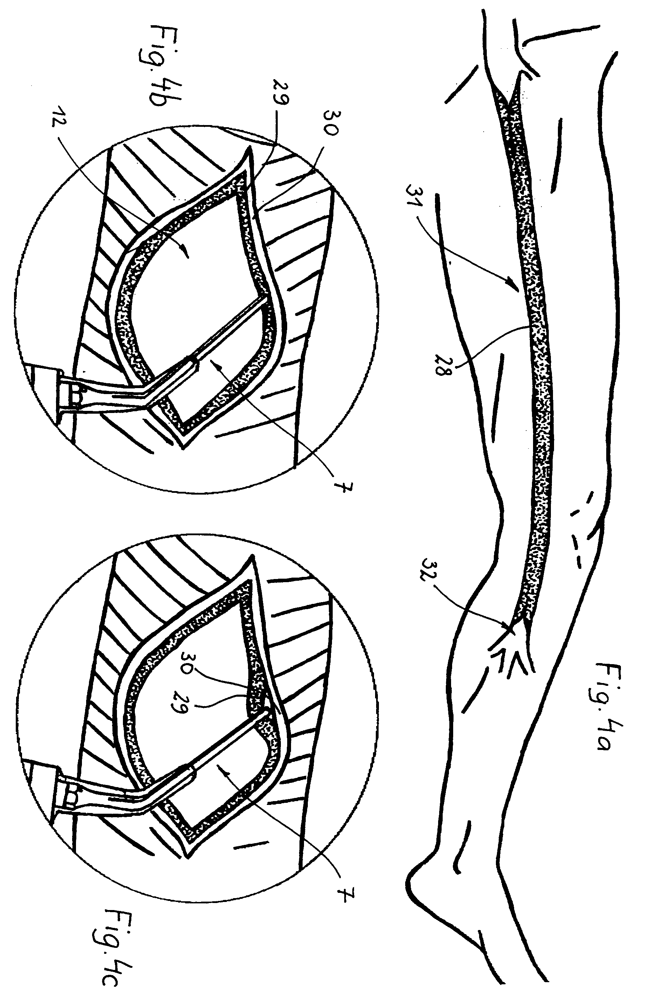

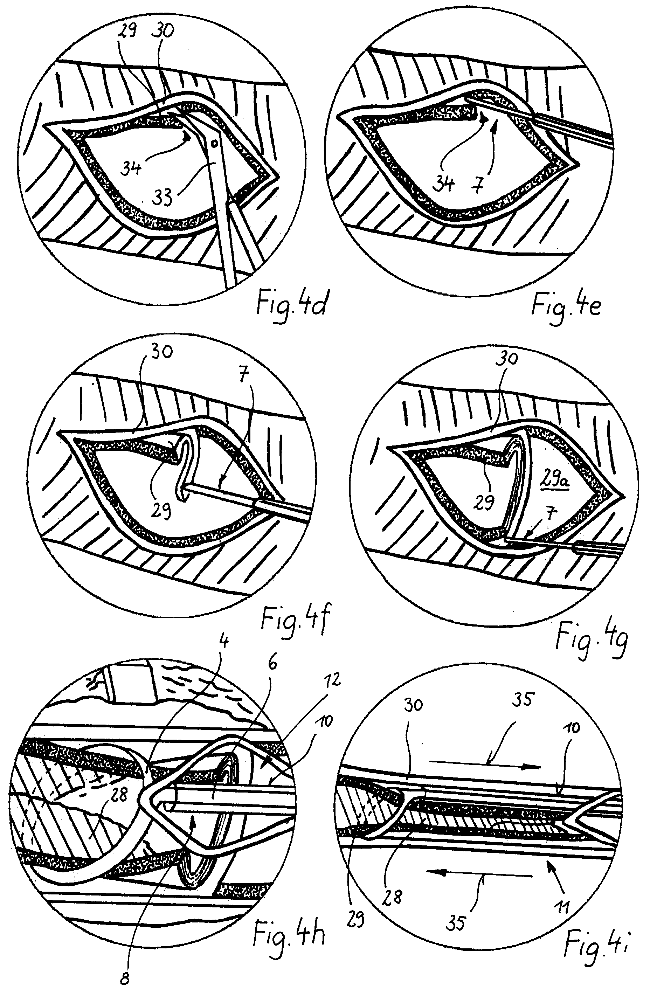

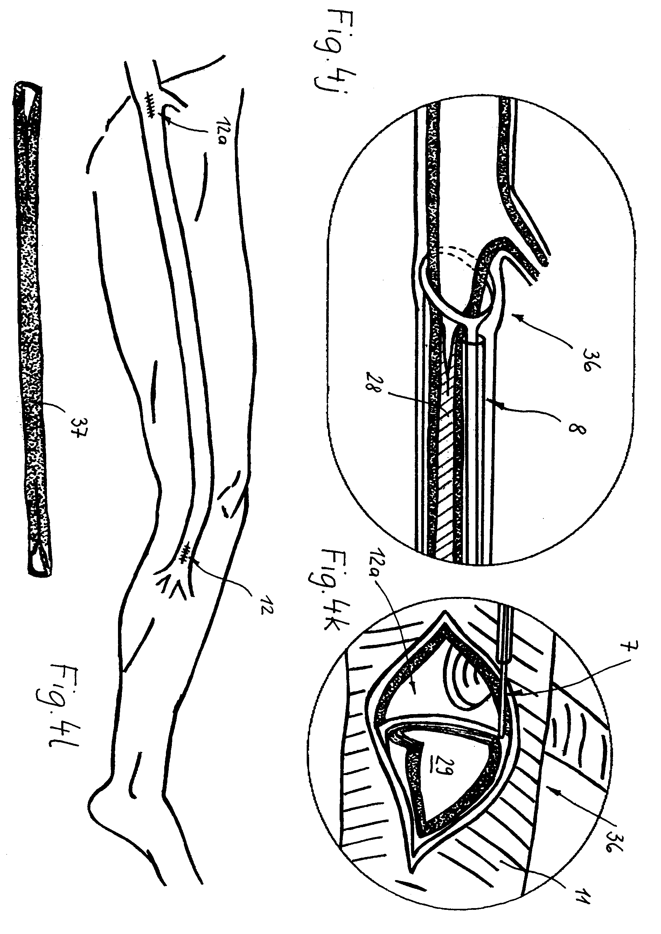

Fig.1 zeigt einen Ultraschall-Generator 17 mit zwei Anschlüssen 18, 19, an den über Leitungen 23 zwei erfindungsgemäße Vorrichtungen zum Ablösen aterosklerotischer Plaques angeschlossen sind. Die beiden Vorrichtungen tragen an ihren jeweiligen Handgriffen 1 Sonden 7 bzw. 8 unterschiedlicher Ausgestaltung, wie besser aus Fig.2 bzw. 3 und 3a zu ersehen ist und deren spezifischer Einsatz anhand der Fig.4a bis 4l beschrieben wird. In den Handgriffen 1 sind piezoelektrische Ultraschall-Transmitter vorgesehen, die über die Output-Signale des Generators mit entsprechender Frequenz und Leistung angeregt werden.

Sterile Flüssigkeit wird aus einem Flüssigkeitsbehälter 20 mittels einer peristaltischen Pumpe 21 und über eine Flüssigkeitsleitung 22 zu in den jeweiligen Handgriffen 1 der beiden Vorrichtungen vorgesehenen Flüssigkeitskanälen 2 (Fig.2 bzw. 3) geleitet. Ein T-Hahn 24 ist dazu vorgesehen, die Flüssigkeit jeweils der Sonde, die gerade in Verwendung ist, zuzuleiten. Flüssigkeitszuleitungen und Leitungen 23 sind dabei bereichsweise gemeinsam geführt.

Über einen Pedalschalter 25 können die jeweils einzusetzenden Vorrichtungen angewählt und die Zuleitung der Flüssigkeit mit Aktivierung der Pumpe 21 in Gang gesetzt werden. Auf einem Display 26 werden alle notwendigen Daten, wie angewählte Vorrichtung, Ultraschall-Frequenz oder Zeitdauer der Behandlung angezeigt.

Fig.2 zeigt die Sonde 8, die zum Einführen in einen in einer Arterie angebrachten Einschnitt geeignet ist. Eine solche Sonde 8 wird im folgenden als Ringsonde bezeichnet, da ein als Ring ausgebildeter, sogenannter Arbeitsteil 4 an einem langen, stabförmigen Übertragungsteil 6 leicht geknickt gegen die Längsachse 13 des Übertragungungsteils 6 angelenkt ist. Der Übertragungsteil 6 ist an dem den Ultraschall-Transmitter umfassenden Handgriff 1 befestigt, in dessen Innerem der Kanal 2 für die sterile Flüssigkeit vorgesehen ist, die daraus aus einer Öffnung 27 in das Innere eines Raumes gedrückt wird, der durch ein den Übertragungsteil 6 umhüllendes Röhrchen 10 begrenzt ist. Das Röhrchen 10 ist aus an sich festem, aber doch in der Längserstreckung der Sonde 8 flexiblem Kunststoff, vorzugsweise PTFE, der neben diesen Eigenschaften eine besonders gute Gleitfähigkeit besitzt. Dies wird anhand der in den Fig.4a bis 4l beschriebenen Arbeitsweise deutlich.

Das Röhrchen 10 ist an seinem dem Arbeitsteil 4 zugewandten Ende offen, sodaß die Flüssigkeit, die über die Pumpe 21 (Fig.1) in den Kanal 2 gedrückt, aus dieser kreisrunden Öffnung 15 mit einem bestimmten Druck austritt, und damit den ringförmigen Arbeitsteil allseits benetzt. Der Arbeitsteil 4 führt im wesentlichen transversale Schwingungen aus, in der Flüssigkeit bilden sich Kavitations-Hohlräume, Drücke von 2 bis 3 atm werden beim Zusammenstürzen dieser Hohlräume frei und wirken auf das umgebende Material. Derartige Ringsonden 8 werden je nach innerem Arteriendurchmesser verschieden große Ringdurchmesser besitzen.

Fig.3 und 3a zeigen eine kurze Sonde 7, die sich zur Entfernung von kleinflächigen Plaques und insbesondere zur Präparation des sogenannten "end point" bzw. des "proximal point" eignet. Der Arbeitsteil 3 dieser Sonde 7 ist spatelförmig ausgebildet, ein Bereich des Übertragungsteils 6 ist gegenüber der durch die gemeinsame Achse von Handgriff 1 und daran anschließenden Übertragungsteil bestimmten Achse 14 abgeknickt ausgebildet. Der Übertragungsteil 5 ist vollständig von einem Flüssigkeit führenden Röhrchen 9 umhüllt. Die Flüssigkeit wird wie bei der Ringsonde 8 (Fig.2) über die Pumpe 21 (Fig.1) über den Kanal 2 im Handgriff 1 aus der Öffnung 27 in den Zwischenraum zwischen Röhrchen 9 und Übertragungsteil 5 gedrückt. Der spatelförmige Arbeitsteil 3 ragt frei aus dem Röhrchen 9 heraus, das in diesem Bereich des Übergangs zwischen Arbeitsteil 3 und Übertragungsteil 5 an der Sonde 7 fest anliegt. Zwei Öffnungen 16 (Detail A der Fig.3a) sind beidseitig des spatelförmigen Arbeitsteils 3 vorgesehen, durch die die Flüssigkeit unter Druck auf den Arbeitsteil 3 gedrückt wird, womit - auch bei schwingendem Arbeitsteil - kein sofortiges Abspritzen der Flüssigkeit im Bereich der Öffnung(en) erfolgen kann (wie beispielsweise bei der AT-A-340 572 der Fall), es erfolgt eine gleichmässige, allseitige Benetzung des Arbeitsteils.

Eine solche kurze Sonde kann gegebebenfalls auch anders ausgebildet sein, je nach erforderlichem Einsatz. Der Übertragungsteil kann ebenso wie bei der Ringsonde gerade ausgebildet sein, der Arbeitsteil kann beispielsweise stäbchen- oder linsenkopfförmig ausgeformt sein. Danach wird auch die Art und Anzahl der Öffnungen für den Austritt der Flüssigkeit aus dem Röhrchen zu wählen sein. Wesentlich ist, daß die Öffnungen im wesentlichen symmetrisch um den Umfang des Arbeitsteils herum angeordnet sind.

Das Röhrchen bei der sogenannten Spatelsonde 7, die im allgemeinen nicht in das Innere einer Arterie eingeführt wird, bzw. nur mit ihrem Arbeitsteil 3 - wie aus den Fig.4a bis 4l zu ersehen ist -, ist aus Kunststoff, wobei sich beispielsweise PVC aufgrund seiner vorteilhaften Eigenschaften, wie bereits einleitend dargestellt, anbietet.

Die erfindungsgemäße Vorrichtung kann für Disobliterationen in den unterschiedlichsten Gefäßen eingesetzt werden, sei es bei Koronarsklerosen oder der Arteriosklerose des Subklavia-Anfangsteils. Anhand der Disobliteration eines peripheren Gefäßes, nämlich der Arteria femoralis 31, die gegebenenfalls langausgedehnte aterosklerotische Obliterationen haben kann, wird in den Fig.4a bis 4l der Einsatz der erfindungsgemäßen Vorrichtung beschrieben.

Nachdem, beispielsweise röntgenographisch die Ausdehnung der Plaque-Obliteration 38 in der Arteria femoralis 31 festgestellt wurde (Fig.4a), wird die Arterie 31 im Bereich des sogenannten "end point" 32 freigelegt und ein Einschnitt 12 gemacht. Dieser Einschnitt 12 ist ca 1 bis 1,5 cm lang. Um die Gefahr von postoperativen Thrombosen klein zu halten, muß die Präparierung des "end point" 32 so erfolgen, daß der Übergang zwischen behandelter und unbehandelter Arterieninnenwand glatt, d.h. ohne wegstehende Gewebefetzchen oder -ränder ist.

Der Einschnitt 12 liegt im Bereich des "end point" 32 und damit an einer Stelle, an der keine Obliterationen mehr vorhanden sind. Die vibrierende Spatelsonde 7 wird an die Gefäßwand geführt (Fig.4b), aufgrund des Kavitationsdrucks von ca 2 bis 3 atm auf das Gewebe löst sich die Media 29 von der Adventitia 30, da die Verbindung zwischen diesen beiden Arterien-Wandschichten relativ schwach ist (Fig.4c). Diese relativ schwache Verbindung zwischen der nicht mit Plaque behafteten Media und der Adventitia war bei den bekannten Methoden gerade mit ein Grund für die teils grob unregelmässigen Oberflächen der verbleibenden Arterieninnenwand bei Präparierung des "end-point".

Es ist festzuhalten, daß allein durch das Heranführen der abgerundeten Spitze der vibrierenden Spatelsonde 7 an die Wandschicht ein - zwar nur geringfügiges - Lösen der Media 29 von der Adventitia 30 stattfindet (Fig.4c), so daß danach mittels einer kleinen Zange 33 ein Transversal-Einschnitt 34 von ca 0,5 bis 1 mm Länge in die Media 29 gemacht werden kann (Fig.4d).

In diesen kleinen Transversal-Einschnitt 34 wird die Rundspitze der Spatelsonde 7 eingeführt (Fig.4e), und transversal rund um die Innenwandung der Arterie geführt (Fig.4f,4g). Dabei findet aufgrund des kavitationalen Druckes eine regelmäßige und glatte Trennung der kreisförmig an der Gefäßinnenwand anliegenden Muskelfasern und der Intima statt, ohne daß ein Schnitt vorgenommen wird. Damit ist die Präparierung des "end-point" erreicht, ohne andere Behandlungsschritte, wie gegebenenfalls das Anbringen von Nahtstichen im Inneren der Arterie. Fig.4g zeigt die glatte Trennung zwischen der von der Adventitia 30 gelösten Media-Schicht 29 und der an der Adventitia 30 haftenden Media-Schicht 29a.

Die zweite Behandlungsphase bezieht sich auf die totale Disobstruktion des die Arterie okkludierenden Zylinders. Dazu wird die Ringsonde 8 in den Einschnitt 12 eingeschoben, so daß sich der vibrierende, ringförmige Arbeitsteil 4 zwischen abgelöster Media 29 und Adventitia 30 befindet (Fig.4h). In Abhängigkeit vom Lumen der Arterie wird der Durchmesser des Rings 4 der Ringsonde 8 gewählt werden. In oben beschriebener Weise, d.h. bei einem Druck von 2 bis 3 atm auf die Gewebeschichten, löst sich die Media 29 und damit auch die obstruierende Plaque 28 von der Adventitia 30, ohne daß es zu einer traumatisierenden Einwirkung auf die Gefäßwand kommt. Da das den vibrierenden Übertragungsteil 6 umhüllende Röhrchen 10 aus einem Material mit hohem Gleitkoeffizienten ist, geschieht das weitere Vorschieben innerhalb der Arterie 11 ungehindert, die Schwingungsfähigkeit des Übertragungsteils 6 wird nicht infolge Wanddrucks auf denselben vermindert oder sogar aufgehoben (Fig.4i).

Dieser zweite Behandlungsschritt, bei dem die Ringsonde 8 sanft vor- und gegebenenfalls auch zurückgeschoben wird, entsprechend den Pfeilen 35 in Fig.4i, endet am "proximal point" 36, d.h. an einer Stelle der Arterie, die wieder frei von okkludierenden Plaques 28 ist (Fig.4j).

Dieser "proximal point" 36 wird nun in einem letzten Behandlungsschritt in ganz entsprechender Weise wie der "end point" 32 präpariert. Nach Zurückziehen und Entfernen der Ringsonde 8 durch den Einschnitt 12 am "end point" 32 (Fig.4a, 4b) wird ein Einschnitt 12a in Longitudinalrichtung der Arterie 11 angebracht, mittels der Spatelsonde 7 erst eine kleinflächige Ablösung der Media 29 bewirkt, in anhand der Fig.4d beschriebener Weise ein kleiner Transversal-Einschnitt in die abgelöste Media 29 gemacht und danach die vibrierende Spatelsonde 7 eingeführt und durch drehende Bewegung derselben das Ablösen der Media 29 von der Adventitia 30 bewirkt (Fig.4k). Ebenso wie bei der Präparierung des "end point" ist die Trennlinie zwischen abgelöster und verbleibender Media sauber und glatt, die Gefahr der Entstehung von postoperativen Thrombosen wird minimiert.

Durch einen der beiden Einschnitte 12 oder 12a am "end point" bzw. am "proximal point"kann der gesamte Okklusionszylinder 37 herausgezogen werden, die Einschnitte 12 und 12a werden vernäht und der Blutfluß in dem disostruierten Artereinbereich wird wieder aktiviert (Fig.4l).

Die verbleibende Innenwand der disostruierten Arterie ist frei von Unregelmäßigkeiten, da die gesamte Media in diesem Bereich entfernt wurde, unabhängig von dem jeweiligen Grad der Aterosklerose.

## Patentansprüche

1. Vorrichtung zur Ablösung von aterosklerotischen Plaques (28) mittels Ultraschall-Schwingungen, mit einem an einen Ultraschall-Generator (17) anschließbaren Ultraschall-Transmitter, einer an einem Handgriff (1) befestigten Sonde (7;8), die einen Arbeitsteil (3;4) und einen Übertragungsteil (5;6) aufweist, und mit einem den Übertragungsteil (5;6) vollständig umhüllenden, Flüssigkeit führenden Röhrchen (9;10), welche Flüssigkeit zur Benetzung des Arbeitsteils (3;4) der Sonde (7;8) dient, wobei für die Zuführung der Flüssigkeit in das Röhrchen (9;10) ein Kanal (2) im Handgriff (1) vorgesehen ist, dadurch gekennzeichnet, daß das Röhrchen (9;10) aus einem Material besteht, dessen Reibungskoeffizient gegenüber der Flüssigkeit vernachlässigbar ist und daß Mittel (15;16) zum allseitigen Benetzen des Arbeitsteils (3;4) mit Flüssigkeit vorgesehen sind.

2. Vorrichtung nach Anspruch 1, insbesondere für das Einschieben in einen in einer Arterie (11) angebrachten Einschnitt (12), dadurch gekennzeichnet, daß der Arbeitsteil (4) in Form eines einseitig an dem Übertragungsteil (6) angelenkten Ringes ausgebildet ist und daß vorzugsweise die Fläche des Ringes mit der Längsachse (13) des Übertragungsteils (6) einen Winkel größer Null einschließt.

3. Vorrichtung zur Ablösung von aterosklerotischen Plaques, insbesondere im Nahbereich eines Einschnitts (12) in eine Arterie (11), nach Anspruch 1, dadurch gekennzeichnet, daß der Arbeitsteil (3) stabförmig - insbesondere in Form eines Spatels - ausgebildet ist und vorzugsweise mit der Längsachse (14) des Übertragungsteils (5) - bezogen auf dessen unmittelbar an den Handgriff (1) anschließenden Bereich - einen Winkel größer Null einschließt.

4. Vorrichtung nach einem der vorhergehenden Ansprüche, dadurch gekennzeichnet, daß das Röhrchen (9;10) einenends an dem Handgriff (1) befestigt ist und anderenends wenigstens eine solche Öffnung (15;16) vorgesehen ist, daß die Flüssigkeit zum allseitigen Benetzen des Arbeitsteils (3;4) ausgebracht werden kann.

5. Vorrichtung nach Anspruch 4, dadurch gekennzeichnet, daß das Röhrchen (9) an seinem dem Handgriff (1) abgekehrten Ende den Arbeitsteil (3) im Bereich des Übergangs zum Übertragungsteil (5) umschließt, wobei wenigstens zwei Öffnungen (16) symmetrisch beidseitig des Arbeitsteils (3) vorgesehen sind.

6. Vorrichtung nach einem der vorhergehenden Ansprüche, dadurch gekennzeichnet, daß der Übertragungsteil (6) und das Röhrchen (10) biegsam sind.

7. Vorrichtung nach einem der vorhergehenden Ansprüche, dadurch gekennzeichnet, daß das Röhrchen (9;10) aus Kunststoff, vorzugsweise aus PVC oder PTFE, besteht.

8. Behandlungsset mit einem Ultraschall-Generator (17) dadurch gekennzeichnet, daß dem Ultraschall-Generator (17) folgende Teile zugeordnet sind:
- wenigstens zwei Anschlüsse (18;19) für jeweils wenigstens je eine der Vorrichtungen nach Anspruch 2 bzw. 3, insbesondere in der Ausbildung nach einem der Ansprüche 4 bis 7;
- wenigstens ein den Anschlüssen (18;19) zugeordneter Amplitudentransformator ;
- wenigstens einen Behälter (20) für die Flüssigkeit, mit einer, insbesondere eine Förderpumpe (21) aufweisenden, Zuleitung (22) zu dem Kanal (2) der jeweiligen Vorrichtung.
